# EUROPEAN PATENT SPECIFICATION

(11) **EP 1 819 326 B1**
(45) Date of publication and mention of the grant of the patent: **02.07.2014**
(21) Application number: 05826219.7
(22) Date of filing: 28.11.2005
(51) Int. Cl.: A61K 31/135, A61P 37/06, A61K 31/397

(54) **DOSAGE REGIMEN OF AN S1P RECEPTOR AGONIST**
DOSIERUNGSPLAN EINES S1P-REZEPTORAGONISTEN
POSOLOGIE D'UN AGONISTE DU RECEPTEUR S1P

(30) Priority: 29.11.2004 US 631483 P
(43) Date of publication of application: 22.08.2007
(60) Divisional application: 10179081.4
(73) Proprietor: Novartis AG, 4056 Basel (CH); Novartis Pharma GmbH, 1230 Wien (AT)
(72) Inventor: KOVARIK, John, M., CH-4056 Basel (CH); APPEL-DINGEMANSE, Silke, CH-4123 Allschwil (CH)
(74) Representative: Jeffries, Charles Edward
(86) International application number: PCT/US2005/043044
(87) International publication number: WO 2006/058316

(56) References cited:
- WO-A-02/100148
- WO-A-03/061567
- WO-A-03/062252
- US-A1- 2003 003 099
- SKERJANEC, A. ET AL: "Systemic exposure and preliminary efficacy of FTY720 in de novo renal transplant recipients" AM J TRANSPLANT (SUPPL. 3): ABST 964, vol. 2, 2002, XP002375195 USA

## Description

The present invention, which is defined in the independent and as specific embodiments in the dependent claims referring to the independent claims, relates to an S1P receptor modulator or agonist for use in a method of the treatment patients suffering from autoimmune diseases or disorders using a loading regimen, as well well as to kits for said use. The claims are incorporated into the present description by reference.

S1P receptor modulators or agonists are compounds which signal as agonists at one or more sphingosine-1 phosphate receptors, e.g. S1P1 to S1P8. Agonist binding to a S1P receptor may e.g. result in dissociation of intracellular heterotrimeric G-proteins into Gα-GTP and Gpγ-GTP, and/or increased phosphorylation of the agonist-occupied receptor and activation of downstream signaling pathways/kinases.

S1P receptor modulators or agonists are valuable compounds for the manufacture of medication for the treatment of various conditions in mammals, especially in human beings. For example, efficacy in transplantation has been demonstrated in rats (skin, heart, liver, small bowel), dogs (kidney), and monkeys (kidney) models. Combination experiments with cyclosporin A showed synergy in skin and heart transplantation models in rats and in monkey renal transplantation. S1P receptor agonists or modulators combined with everolimus prolong survival of cardiac (rat) and renal (monkey) allografts. Due to their immune-modulating potency, S1P receptor modulators or agonists are also useful for the treatment of inflammatory and autoimmune diseases. Further characteristics of S1P receptor agonists can be found in the following publications:
Brinkmann V, Chen S, Feng L, et al (2001) FTY720 alters lymphocyte homing and protects allografts without inducing general immunosuppression. Transplant Proc; 33:530-531.
Brinkmann V, Pinschewer D, Feng L, et al (2001) FTY720: altered lymphocyte traffic results in allograft protection (review). Transplantation; 72:764-769.
Pinschewer DD, Ochsenbein AF, Odermatt B, et al (2000) FTY720 immunosuppression impairs effector T-cell peripheral homing without affecting induction, expansion, and memory. J Immunol; 164:5761.
Yanagawa Y, Sugahara K, Kataoka H, et al (1998) FTY720, a novel immunosuppressant, induces sequestration of circulating mature lymphocytes by acceleration of lymphocyte homing in rats. II. FTY720 prolongs skin allograft survival by decreasing T cell infiltration into grafts but not cytokine production in vivo. J Immunol.; 160(11):5493-9.
Skerjanec et al., Am. J. Transplant. (Suppl. 3), Vol. 2, Abstract 964, relates to *de novo* renal transplant patients receiving a single loading dosage of FTY720 during transplantation surgery and in combination with Neoral® and corticosteroids. WO 2003/061567A and WO 2003/062252 refer to S1P modulators different from FTY and provide no loading regimen.

It has now surprisingly been found that a specific dosage regimen will provide further unexpected benefits.

The binding affinity of S1P receptor agonists or modulators to individual human S1P receptors may be determined in following assay:
S1P receptor agonist or modulator activities of compounds are tested on the human S1P receptors S1P₁, S1P₂, S1P₃, S1P₄ and S1P₅. Functional receptor activation is assessed by quantifying compound induced GTP [γ-³⁵S] binding to membrane protein prepared from transfected CHO or RH7777 cells stably expressing the appropriate human S1P receptor. The assay technology used is SPA (scintillation proximity based assay). Briefly, DMSO dissolved compounds are serially diluted and added to SPA- bead (Amersham-Pharmacia) immobilised S1 P receptor expressing membrane protein (10-20µg/well) in the presence of 50 mM Hepes, 100 mM NaCl, 10 mM MgCl₂, 10 µM GDP, 0.1 % fat free BSA and 0.2 nM GTP [γ-³⁵S] (1200 Ci/mmol). After incubation in 96 well microtiterplates at RT for 120 min, unbound GTP [γ-³⁵S] is separated by a centrifugation step. Luminescence of SPA beads triggered by membrane bound GTP [γ-³⁵S] is quantified with a TOPcount plate reader (Packard). EC₅₀s are calculated using standard curve fitting software. In this assay, the S1 P receptor modulators or agonists preferably have a binding affinity to S1P receptor <50 nM.

Preferred S1P receptor agonists or modulators are e.g. compounds which in addition to their S1 P binding properties also have accelerating lymphocyte homing properties, e.g. compounds which elicit a lymphopenia resulting from a re-distribution, preferably reversible, of lymphocytes from circulation to secondary lymphatic tissue, without evoking a generalized immunosuppression. Naive cells are sequestered; CD4 and CD8 T-cells and B-cells from the blood are stimulated to migrate into lymph nodes (LN) and Peyer's patches (PP).

The lymphocyte homing property may be measured in following Blood Lymphocyte Depletion assay:
A S1 P receptor agonist or modulator or the vehicle is administered orally by gavage to rats. Tail blood for hematological monitoring is obtained on day -1 to give the baseline individual values, and at 2, 6, 24, 48 and 72 hours after application. In this assay, the S1P receptor agonist or modulator depletes peripheral blood lymphocytes, e.g. by 50%, when administered at a dose of e.g. < 20 mg/kg.

For reference purposes, S1 P receptor modulators or agonists are typically sphingosine analogues, such as 2-substituted 2-amino- propane-1,3-diol or 2-amino-propanol derivatives, e. g. a compound comprising a group of formula X 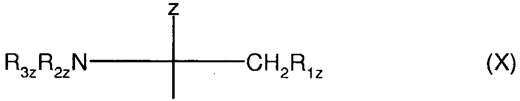 wherein Z is H, C₁₋₆alkyl, C₂₋₆alkenyl, C₂₋₆alkynyl, phenyl, phenyl substituted by OH, C₁₋₆alkyl substituted by 1 to 3 substituents selected from the group consisting of halogen, C₃₈cycloalkyl, phenyl and phenyl substituted by OH, or CH₂-R_{4z} wherein R_{4z} is OH, acyloxy or a residue of formula (a) 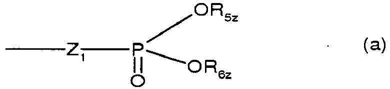
wherein Z₁ is a direct bond or O, preferably O;
each of R_{5z} and R_{6z}, independently, is H, or C₁-₄alkyl optionally substituted by 1, 2 or 3 halogen atoms;
R_{1z} is OH, acyloxy or a residue of formula (a); and each of R_{2z} and R_{3z} independently, is H, C₁₋₄alkyl or acyl.

Group of formula X is a functional group attached as a terminal group to a moiety which may be hydrophilic or lipophilic and comprise one or more aliphatic, alicyclic, aromatic and/or heterocyclic residues, to the extent that the resulting molecule wherein at least one of Z and R_{1z} is or comprises a residue of formula (a), signals as an agonist at one of more sphingosine-1-phosphate receptor.

Examples of appropriate S1 P receptor agonists or modulators are, for example:
- Compounds as disclosed in EP627406A1, e.g. a compound of formula I 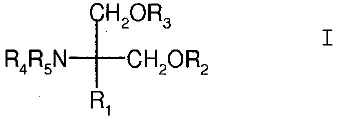 wherein R₁ is a straight- or branched (C₁₂₋₂₂) chain
   - which may have in the chain a bond or a hetero atom selected from a double bond, a triple bond, O, S, NR₆, wherein R₆ is H, C₁₋₄alkyl, aryl-C₁₋₄alkyl, acyl or (C₁₋₄alkoxy)carbonyl, and carbonyl, and/or
   - which may have as a substituent C₁₋₄alkoxy, C₂₋₄alkenyloxy, C₂₋₄alkynyloxy, arylC₁₋₄alkyloxy, acyl, C₁₋₄alkylamino, C₁₋₄alkylthio, acylamino, (C₁₋₄alkoxy)carbonyl, (C₁₋₄alkoxy)-carbonylamino, acyloxy, (C₁₋₄alkyl)carbamoyl, nitro, halogen, amino, hydroxyimino, hydroxy or carboxy; or
   R₁ is
   - a phenylalkyl wherein alkyl is a straight- or branched (C₆₋₂₀)carbon chain; or
   - a phenylalkyl wherein alkyl is a straight- or branched (C₁₋₃₀)carbon chain wherein said phenylalkyl is substituted by
   - a straight- or branched (C₆₋₂₀)carbon chain optionally substituted by halogen,
   - a straight- or branched (C₆₋₂₀)alkoxy chain optionally substitued by halogen,
   - a straight- or branched (C₆₋₂₀)alkenyloxy,
   - phenyl-C₁₋₁₄alkoxy, halophenyl-C₁₋₄alkoxy, phenyl-C₁₋₁₄alkoxy-C₁₋₁₄alkyl, phenoxy-C₁₋₄alkoxy or phenoxy-C₁₋₄alkyl,
   - cycloalkylalkyl substituted by C₆₋₂₀alkyl,
   - heteroarylalkyl substituted by C₆₋₂₀alkyl,
   - heterocyclic C₆₋₂₀alkyl or
   - heterocyclic alkyl substituted by C₂₋₂₀alkyl ,
   and wherein
   the alkyl moiety may have
   - in the carbon chain, a bond or a heteroatom selected from a double bond, a triple bond, O, S, sulfinyl, sulfonyl, or NR₆, wherein R₆ is as defined above, and
   - as a substituent C₁₋₄alkoxy, C₂₋₄alkenyloxy, C₂₋₄alkynyloxy, arylC₁₋₄alkyloxy, acyl, C₁₋₄alkylamino, C₁₋₄alkylthio, acylamino, (C₁₋₄alkoxy)carbonyl, (C₁₋₄alkoxy)carbonylamino, acyloxy, (C₁₋₄alkyl)carbamoyl, nitro, halogen, amino, hydroxy or carboxy, and
   each of R₂, R₃, R₄ and R₅, independently, is H, C₁₋₄alkyl or acyl
   or a pharmaceutically acceptable salt or hydrate thereof;
   - Compounds as disclosed in EP 1002792A1, e.g. a compound of formula II 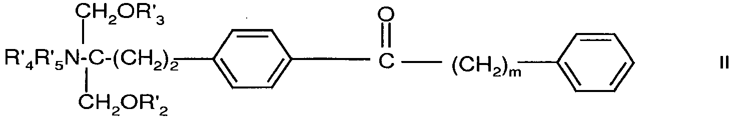
   wherein m is 1 to 9 and each of R'₂, R'₃, R'₄ and R'₅, independently, is H, C₁₋₆alkyl or acyl, or a pharmaceutically acceptable salt or hydrate thereof;
- Compounds as disclosed in EP0778263 A1, e.g. a compound of formula III 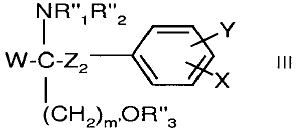
   wherein W is H; C₁₋₆alkyl, C₂₋₆alkenyl or C₂₋₆alkynyl; unsubstituted or by OH substituted phenyl; R"₄O(CH₂)ₙ; or C₁₋₆alkyl substituted by 1 to 3 substituents selected from the group consisting of halogen, C₃₋₈cycloalkyl, phenyl and phenyl substituted by OH;
   X is H or unsubstituted or substituted straight chain alkyl having a number p of carbon atoms or unsubstituted or substituted straight chain alkoxy having a number (p-1) of carbon atoms, e.g. substituted by 1 to 3 substitutents selected from the group consisting of C₁₋₆alkyl, OH, C₁-₆alkoxy, acyloxy, amino, C₁₋₆alkylamino, acylamino, oxo, haloC₁₋₆alkyl, halogen, unsubstituted phenyl and phenyl substituted by 1 to 3 substituents selected from the group consisting of C₁₆alkyl, OH, C₁₋₆alkoxy, acyl, acyloxy, amino, C₁₋₆alkylamino, acylamino, haloC₁₋₆alkyl and halogen; Y is H, C₁₋₆alkyl, OH, C₁₋₆alkoxy, acyl, acyloxy, amino, C₁₋₆alkylamino, acylamino, haloC₁₋₆alkyl or halogen, Z₂ is a single bond or a straight chain alkylene having a number or carbon atoms of q,
   each of p and q, independently, is an integer of 1 to 20, with the proviso of 6≤p+q≤23, m' is 1, 2 or 3, n is 2 or 3,
   each of R"₁, R"₂, R"₃ and R"₄, independently, is H, C₁₋₄alkyl or acyl,
   or a pharmaceutically acceptable salt or hydrate thereof,
- Compounds as disclosed in WO02/18395, e.g. a compound of formula IVa or IVb 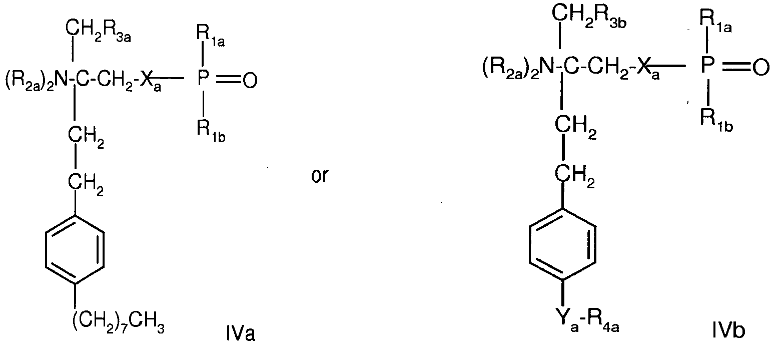 wherein Xₐ is O, S, NR₁ₛ or a group -(CH₂)ₙₐ-, which group is unsubstituted or substituted by 1 to 4 halogen; nₐ is 1 or 2, R₁ₛ is H or (C₁₋₄)alkyl, which alkyl is unsubstituted or substituted by halogen; R₁ₐ is H, OH, (C₁₋₄)alkyl or O(C₁₋₄)alkyl wherein alkyl is unsubstituted or substituted by 1 to 3 halogen; R_{1b} is H, OH or (C₁₋₄)alkyl, wherein alkyl is unsubstituted or substituted by halogen; each R₂ₐ is independently selected from H or (C₁₋₄)alkyl, which alkyl is unsubstituted or substitued by halogen; R₃ₐ is H, OH, halogen or O(C₁₋₄)alkyl wherein alkyl is unsubstituted or substituted by halogen; and R_{3b} is H, OH, halogen, (C₁₋₄)alkyl wherein alkyl is unsubstituted or substituted by hydroxy, or O(C₁₋₄)alkyl wherein alkyl is unsubstituted or substituted by halogen; Yₐ is -CH₂-, -C(O)-, -CH(OH)-, -C(=NOH)-, O or S, and R₄ₐ is (C₄₋₁₄)alkyl or (C₄₋₁₄)alkenyl;
   or a pharmaceutically acceptable salt or hydrate thereof;
- Compounds as disclosed in WO 02/076995, e.g. a compound of formula V 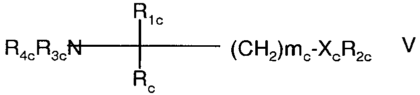 wherein
   - m_{c}: is 1, 2 or 3;
   - X_{c}: is O or a direct bond;
   - R_{1c}: is H; C₁₋₆alkyl optionally substituted by OH, acyl, halogen, C₃₋₁₀cycloalkyl, phenyl or hydroxy-phenylene; C₂₋₆alkenyl; C₂₋₆alkynyl; or phenyl optionally substituted by OH;
   - R_{2c}: is 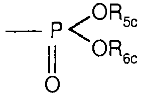 wherein R_{5c} is H or C₁₋₄alkyl optionally substituted by 1, 2 or 3 halogen atoms, and R_{6c} is H or C₁₋₄alkyl optionally substituted by halogen;
   each of R_{3c} and R_{4c}, independently, is H, C₁₋₄alkyl optionally substituted by halogen, or acyl, and
   - R_{c}: is C₁₃₋₂₀alkyl which may optionally have in the chain an oxygen atom and which may optionally be substituted by nitro, halogen, amino, hydroxy or carboxy; or a residue of formula (a) 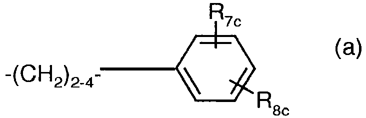 wherein R_{7c} is H, C₁₋₄alkyl or C₁₋₄alkoxy, and R_{8c} is substituted C₁₋₂₀alkanoyl, phenylC₁₋₁₄alkyl wherein the C₁₋₁₄alkyl is optionally substituted by halogen or OH, cycloalkylC₁₋₁₄alkoxy or phenylC₁₋₁₄alkoxy wherein the cycloalkyl or phenyl ring is optionally substituted by halogen, C₁₋₄alkyl and/or C₁₋₄alkoxy, phenylC₁₋₄alkoxy-C₁₋₁₄alkyl, phenoxyC₁₋₁₄alkoxy or phenoxyC₁₋₁₄alkyl,
   - R_{c}: being also a residue of formula (a) wherein R_{8c} is C₁₋₁₄alkoxy when R_{1c} is C₁₋₄alkyl, C₂₋₆alkenyl or C₂₋₆alkynyl,
   or a compound of formula VI 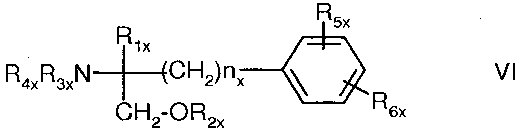 wherein
   nₓ is 2, 3 or 4
   R₁ₓ is H; C₁₋₆alkyl optionally substituted by OH, acyl, halogen, cycloalkyl, phenyl or hydroxy-phenylene; C₂₋₆alkenyl; C₂₋₆alkynyl; or phenyl optionally substituted by OH;
   R₂ₓ is H, C₁₋₄alkyl or acyl
   each of R₃ₓ and R₄ₓ, independently is H, C₁₋₄alkyl optionally substituted by halogen or acyl,
   R₅ₓ is H, C₁₋₄alkyl or C₁₋₄alkoxy, and
   R₆ₓ is C₁₋₂₀ alkanoyl substituted by cycloalkyl; cyloalkylC₁₋₁₄alkoxy wherein the cycloalkyl ring is optionally substituted by halogen, C₁₋₄alkyl and/or C₁₋₄alkoxy; phenylC₁₋₁₄alkoxy
   wherein the phenyl ring is optionally substituted by halogen, C₁₋₄alkyl and/or C₁₋₄alkoxy, R₆ₓ being also C₄₋₁₄alkoxy when R₁ₓ is C₂₋₄alkyl substituted by OH, or pentyloxy or hexyloxy when R₁ₓ is C₁₋₄akyl,
   provided that R₆ₓ is other than phenyl-butylenoxy when either R₅ₓ is H or R₁ₓ is methyl,
   or a pharmaceutically acceptable salt or hydrate thereof;
- Compounds as disclosed in WO02/06268A1, e.g. a compound of formula VII 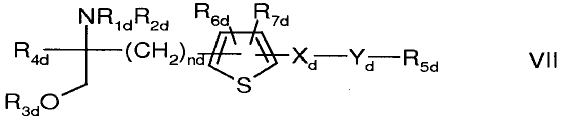 wherein each of R_{1d} and R_{2d}, independently, is H or an amino-protecting group; R_{3d} is hydrogen, a hydroxy-protecting group or a residue of formula 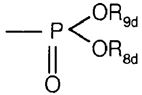
   R_{4d} is C₁₋₄alkyl ;
   n_{d} is an integer of 1 to 6;
   X_{d} is ethylene, vinylene, ethynylene, a group having a formula - D-CH₂- (wherein D is carbonyl, - CH(OH)-, O, S or N), aryl or aryl substituted by up to three substitutents selected from group a as defined hereinafter;
   Y_{d} is single bond, C₁₋₁₀alkylene, C₁₋₁₀alkylene which is substituted by up to three substitutents selected from groups a and b, C₁₋₁₀alkylene having O or S in the middle or end of the carbon chain, or C₁₋₁₀alkylene having O or S in the middle or end of the carbon chain which is substituted by up to three substituents selected from groups a and b;
   R_{5d} is hydrogen, C₃₋₆cycloalkyl, aryl, heterocyclic group, C₃₋₆cycloalkyl substituted by up to three substituents selected from groups a and b, aryl substituted by up to three substituents selected from groups a and b, or heterocyclic group substituted by up to three substituents selected from groups a and b;
   each of R_{6d} and R_{7d}, independently, is H or a substituent selected from group a;
   each of R_{8d} and R_{9d}, independently, is H or C₁₋₄alkyl optionally substituted by halogen;
   <group a > is halogen, lower alkyl, halogeno lower alkyl, lower alkoxy, lower alkylthio, carboxyl, lower alkoxycarbonyl, hydroxy, lower aliphatic acyl, amino, mono-lower alkylamino, di-C₁₋₄alkylamino, acylamino, cyano or nitro; and
   <group b > is C₃₋₆cycloalkyl, aryl or heterocyclic group, each being optionally substituted by up to three substituents selected from group a;
   with the proviso that when R_{5d} is hydrogen, Y_{d} is a either a single bond or linear C₁₋₁₀ alkylene, or a pharmacologically acceptable salt, ester or hydrate thereof;
- Compounds as disclosed in JP-14316985 (JP2002316985), e.g. a compound of formula VIII 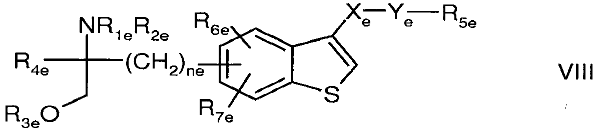 wherein R₁ₑ,R₂ₑ,R₃ₑ,R₄ₑ,R₅ₑ,R₆ₑ,R₇ₑ, nₑ, Xₑ and Yₑ are as disclosed in JP-14316985; or a pharmacologically acceptable salt, ester or hydrate thereof;
- Compounds as disclosed in WO 03/29184 and WO 03/29205, e.g. compounds of formula IX 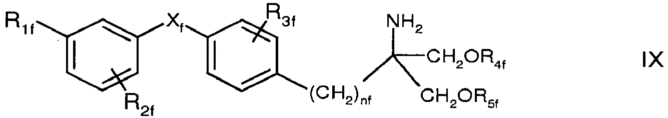
   wherein X_{f} is O, S, SO or SO₂
   R_{1f} is halogen, trihalomethyl, OH, C₁₋₇alkyl, C₁₋₄alkoxy, trifluoromethoxy, phenoxy, cyclohexylmethyloxy, pyridylmethoxy, cinnamyloxy, naphthylmethoxy, phenoxymethyl, CH₂-OH, CH₂-CH₂-OH, C₁₋₄alkylthio, C₁₋₄alkylsulfinyl, C₁₋₄alkylsulfonyl, benzylthio, acetyl, nitro or cyano, or phenyl, phenylC₁₋₄alkyl or phenyl-C₁₋₄alkoxy each phenyl group thereof being optionally substituted by halogen, CF₃, C₁₋₄alkyl or C₁₋₄alkoxy;
   R_{2f} is H, halogen, trihalomethyl, C₁₋₄alkoxy, C₁₋₇alkyl, phenethyl or benzyloxy;
   R_{3f} H, halogen, CF₃, OH, C₁₋₇alkyl, C₁₋₄alkoxy, benzyloxy or C₁₋₄alkoxymethyl;
   each of R_{4f} and R_{5f}, independently is H or a residue of formula 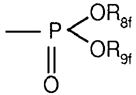
      wherein each of R_{8f} and R_{9f}, independently, is H or C₁₋₄alkyl optionally substituted by halogen;and
      n_{f} is an integer from 1 to 4;
      e.g. 2-amino-2-[4-(3-benzyloxyphenoxy)-2-chlorophenyl]ethyl-1,3-propane-diol, 2-amino-2-[4-(benzyloxyphenylthio)-2- chlorophenyl]ethyl-1 ,3-propane-diol, 2-amino-2-[4-(3-benzyloxyphenoxy)-2-chlorophenyl]propyl-1,3-propane-diol or 2-amino-2-[4-(benzyloxyphenylthio)-2- chlorophenyl]propyl-1,3-propane-diol,
      or a pharmacological salt, solvate or hydrate thereof;
- Compounds as disclosed in WO03/062252A1, e.g. a compound of formula X 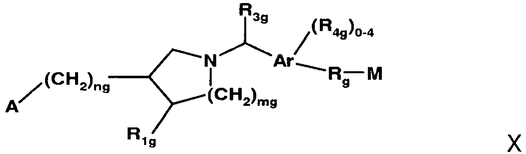 wherein
   Ar is phenyl or naphthyl; each of m_{g} and n_{g} independently is 0 or 1; A is selected from COOH, PO₃H₂, PO₂H, SO₃H, PO(C₁₋₃alkyl)OH and 1 H-tetrazol-5-yl; each of R_{1g} and R_{2g} independently is H, halogen, OH, COOH or C₁₋₄alkyl optionally substituted by halogen; R_{3g} is H or C₁₋₄alkyl optionally substituted by halogen or OH; each R_{4g} independently is halogen, or optionally halogen substituted C₁₋₄alkyl or C₁₋₃alkoxy; and each of R₉ and M has one of the significances as indicated for B and C, respectively, in WO03/062252A1;
   or a pharmacologically acceptable salt, solvate or hydrate thereof;
- Compounds as disclosed in WO 03/062248A2, e.g. a compound of formula XI 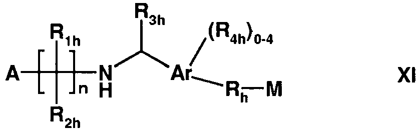 wherein Ar is phenyl or naphthyl; n is 2,3 or 4; A is COOH, 1 H-tetrazol-5-yl, PO₃H₂, PO₂H₂, - SO₃H or PO(R₅ₕ)OH wherein R₅ₕ is selected from C₁₋₄alkyl, hydroxyC₁₋₄alkyl, phenyl, -CO-C₁-₃alkoxy and -CH(OH)-phenyl wherein said phenyl or phenyl moiety is opitonally substituted; each of R₁ₕ and R₂ₕ independently is H, halogen, OH, COOH, or optionally halogeno substituted C₁₋₆alkyl or phenyl; R₃ₕ is H or C₁₋₄alkyl optionally substituted by halogen and/ OH; each R₄ₕ independently is halogeno, OH, COOH, C₁₋₄alkyl, S(O)_{0,1 or 2}C₁₋₃alkyl, C₁₋₃alkoxy, C₃₆cycloalkoxy, aryl or aralkoxy, wherein the alkyl portions may optionally be substituted by 1-3 halogens; and each of Rₕ and M has one of the significances as indicated for B and C, respectively, in WO03/062248A2;
   or a pharmacologically acceptable salt, solvate or hydrate thereof;
- Compounds as disclosed in WO 04/103306A, WO 05/000833, WO 05/103309 or WO 05/113330, e.g. compounds of formula XIIa or XIIb 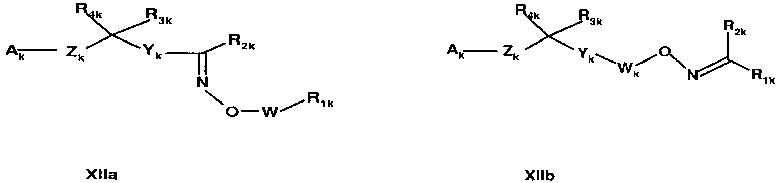 wherein
   Aₖ is COOR₅ₖ, OPO(OR₅ₖ)₂, PO(OR₅ₖ)₂, SO₂OR₅ₖ, POR₅ₖOR₅ₖ or 1 H-tetrazol-5-yl, R₅ₖ being H or C₁₋₆alkyl;
   Wₖ is a bond, C₁₋₃alkylene or C₂₋₃alkenylene;
   Yₖ is C₆₋₁₀aryl or C₃₋₉heteroaryl, optionally substituted by 1 to 3 radicals selected from halogene, OH, NO₂, C₁₋₆a[kyl, C₁₋₆alkoxy; halo-substituted C₁₋₆alkyl and halo-substituted C₁₋₆alkoxy;
   Zₖ is a heterocyclic group as indicated in WO 04/103306A, e.g. azetidine;
   R₁ₖ is C₆₋₁₀aryl or C₃₋₉heteroaryl, optionally substituted by C₁₋₆alkyl, C₆₋₁₀aryl, C₆₋₁₀arylC₁₋₄alkyl, C₃₋₉heteroaryl, C₃₋₉heteroarylC₁₋₄alkyl, C₃₋₈cycloalkyl, C₃₋₈cycloalkylC₁₋₄alkyl, C₃₋₈heterocy_{d}oalkyl or C₃₋₈heterocycloalkylC₁₋₄alkyl; wherein any aryl, heteroaryl, cycloalkyl or heterocycloalkyl of R₁ₖ may be substituted by 1 to 5 groups selected from halogen, C₁₋₆alkyl, C₁₋₆alkoxy and halo substituted-C₁₋₆alkyl or -C₁₋₆alkoxy;
   R₂ₖ is H, C₁₋₆alkyl, halo substituted C₁₋₆alkyl, C₂₋₆alkenyl or C₂₋₆alkynyl: and
   each of R₃ₖ or R₄ₖ, independently, is H, halogen, OH, C₁₋₆alkyl, C₁₋₆alkoxy or halo substituted C₁₋₆alkyl or C₁₋₆alkoxy;
   and the N-oxide derivatives thereof or prodrugs thereof,
or a pharmacologically acceptable salt, solvate or hydrate thereof.

For reference purposes, a S1 P receptor agonist or modulator for use in a combination of the invention may also be a selective S1 P1 receptor, e.g. a compound which possesses a selectivity for the S1 P1 receptor over the S1 P3 receptor of at least 20 fold, e.g. 100, 500, 1000 or 2000 fold, as measured by the ratio of EC₅₀ for the S1 P1 receptor to the EC₅₀ for the S1P3 receptor as evaluated in a ³⁵S-GTPγS binding assay, said compound having an EC₅₀ for binding to the S1 P1 receptor of 100 nM or less as evaluated by the ³⁵S-GTPγS binding assay. Representative S1 P1 receptor agonists or modulators are e.g. the compounds listed in WO 03/061567, for instance a compound of formula XIII or XIV

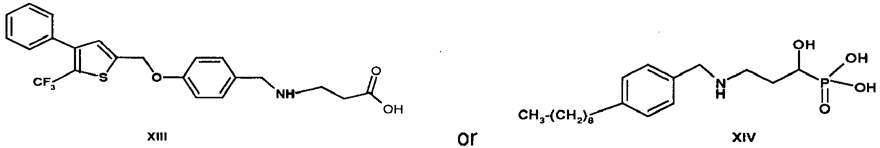

When the compounds of formulae I to XIV have one or more asymmetric centers in the molecule, the present disclosure, for reference, is to be understood as embracing the various optical isomers, as well as racemates, diastereoisomers and mixtures thereof are embraced. Compounds of formula III or IVb, when the carbon atom bearing the amino group is asymmetric, have preferably the R-configuration at this carbon atom.

The compounds of formulae I to XIV may exist in free or salt form. Examples of pharmaceutically acceptable salts of the compounds of the formulae I to XIV include salts with inorganic acids, such as hydrochloride, hydrobromide and sulfate, salts with organic acids, such as acetate, fumarate, maleate, benzoate, citrate, malate, methanesulfonate and benzenesulfonate salts, or, when appropriate, salts with metals such as sodium, potassium, calcium and aluminium, salts with amines, such as triethylamine and salts with dibasic amino acids, such as lysine. The compounds and salts of the combination encompass hydrate and solvate forms.

Acyl as indicated above may be a residue Rγ-CO- wherein Ry is C₁₋₆alkyl, C₃₋₆cycloalkyl, phenyl or phenyl-C₁₋₄alkyl. Unless otherwise stated, alkyl, alkoxy, alkenyl or alkynyl may be straight or branched.

Aryl may be phenyl or naphthyl, preferably phenyl.

When in the compounds of formula I the carbon chain as R₁ is substituted, it is preferably substituted by halogen, nitro, amino, hydroxy or carboxy. When the carbon chain is interrupted by an optionally substituted phenylene, the carbon chain is preferably unsubstituted. When the phenylene moiety is substituted, it is preferably substituted by halogen, nitro, amino, methoxy, hydroxy or carboxy.

Preferred compounds of formula I are those wherein R₁ is C₁₃₋₂₀alkyl, optionally substituted by nitro, halogen, amino, hydroxy or carboxy, and, more preferably those wherein R₁ is phenylalkyl substituted by C₆₋₁₄-alkyl chain optionally substituted by halogen and the alkyl moiety is a C₁₋₆alkyl optionally substituted by hydroxy. More preferably, R₁ is phenyl-C₁₋₆alkyl substituted on the phenyl by a straight or branched, preferably straight, C₆₋₁₄alkyl chain. The C₆₋₁₄alkyl chain may be in ortho, meta or para, preferably in para.

Preferably each of R₂ to R₅ is H.

In the above formula of VII "heterocyclic group" represents a 5- to 7 membered heterocyclic group having 1 to 3 heteroatoms selected from S, O and N. Examples of such heterocyclic groups include the heteroaryl groups indicated above, and heterocyclic compounds corresponding to partially or completely hydrogenated heteroaryl groups, e.g. furyl, thienyl, pyrrolyl, azepinyl, pyrazolyl, imidazolyl, oxazolyl, isoxazolyl, thiazolyl, isothiazolyl, 1,2,3-oxadiazolyl, triazolyl, tetrazolyl, thiadiazolyl, pyranyl, pyridyl, pyridazinyl, pyrimidinyl, pyrazinyl, tetrahydropyranyl, morpholinyl, thiomorpholinyl, pyrrolidinyl, pyrrolyl, imidazolidinyl, pyrazolidinyl, piperidinyl, piperazinyl, oxazolidinyl, isoxazolidinyl, thiazolidinyl or pyrazolidinyl. Preferred heterocyclic groups are 5-or 6-membered heteroaryl groups and the most preferred heteocyclic group is a morpholinyl, thiomorpholinyl or piperidinyl group.

A reference compound of formula I is 2-amino-2-tetradecyl-1,3-propanediol. A particularly preferred S1 P receptor agonist of formula I for use or in the kits for use according to the invention, respectively, is FTY720, i.e. 2-amino-2-[2-(4-octylphenyl) ethyl]propane-1 ,3-diol in free form or in a pharmaceutically acceptable salt form (referred to hereinafter as Compound A), e.g. the hydrochloride, as shown:

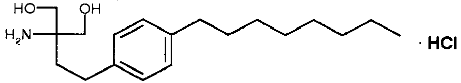

A reference compound of formula II is the one wherein each of R'₂ to R'₅ is H and m is 4, i.e. 2-amino-2-{2-[4-(1-oxo-5-phenylpentyl)phenyl}ethyl}propane-1,3-diol, in free form or in pharmaceutically acceptable salt form (referred to hereinafter as Compound B), e.g the hydrochloride.

A reference compound of formula III is the one wherein W is CH₃, each of R"₁ to R"₃ is H, Z₂ is ethylene, X is heptyloxy and Y is H, i.e. 2-amino-4-(4-heptyloxyphenyl)-2-methyl-butanol, in free form or in pharmaceutically acceptable salt form (referred to hereinafter as Compound C), e.g. the hydrochloride. The R-enantiomer is particularly preferred.

A compound for use or in the kits for use according to the invention, respectively, of formula IVa is the FTY720-phosphate (R₂ₐ is H, R₃ₐ is OH, Xₐ is O, R₁ₐ and R_{1b} are OH). A reference compound of formula IVb is the Compound C-phosphate (R₂ₐ is H, R_{3b} is OH, Xₐ is O, R₁ₐ and R_{1b} are OH, Yₐ is O and R₄ₐ is heptyl). A reference compound of formula V is Compound B-phosphate.

A reference compound of formula V is phosphoric acid mono-[(R)-2-amino-2-methyl-4-(4-pentyloxy-phenyl)-butyl]ester.

A reference compound of formula VIII is (2R)-2-amino-4-[3-(4-cyclohexyloxybutyl)-benzo[b]th ien-6-yl]-2-methylbutan-1-ol.

A reference compound of formula XIIa is e.g. 1-{4-[1-(4-cyclohexyl-3-trifluoromethyl-benzyloxyimino)-ethyl]-2-ethyl-benzyl}-azetidine-3-carboxylic acid, or a prodrug thereof.

For reference purposes, the present disclosure provides the use of an S1 P receptor modulator or agonist in the manufacture of a medication, whereby said medication is administered in such a way that during the initial 3 to 6 days, preferably 4 or 5 days, most preferred 4 days, of treatment the dosage of said S1 P receptor modulator or agonist is raised so that in total the R-fold (R being the accumulation factor) standard daily dosage of said S1 P receptor modulator or agonist is administered and thereafter the treatment is continued with the standard or a lower daily dosage of said S1 P receptor modulator or agonist.

Preferred medications comprise medication for transplant patients providing prolonged survival rates, in particular prolonged allograft survival rates especially for renal, heart, lung or liver transplants, or (according to the invention for the compounds for use or kits for use as claimed, respectively) for patients suffering from autoimmune diseases, e.g. multiple sclerosis, lupus nephritis, rheumatoid arthritis, inflammatory bowel diseases or psoriasis.

In view of the normally prolonged taking of the medication, the standard daily dosage (also called maintenance dose) refers to the dosage of an S1 P receptor modulator or agonist as defined in claims 1 necessary for a steady-state trough blood level of the medication or its active metabolite(s) providing effective treatment. Said dosage is dependent on the accumulation factor (R). By blood level is meant the concentration of a drug in blood at any time. Trough blood level corresponds to a pre-dose blood level. Steady-state means whether the trough or blood level is stable over time. Steady-state trough blood levels may be assessed, for example, by obtaining a pre-dose blood sample anytime after month 3. The accumulation factor (R) is calculated on the ratio of the steady-state trough to the trough just before the second dose.

The dosage of the S1 P receptor modulator or agonist for use or in kits for use according to the invention, respectuively, during the initial 3 to 6 days of treatment is increased stepwise as defined in the claims. Thereafter the treatment is continued with the maintenance therapy with the standard daily dosage or with a lower daily dosage. When the treatment is continued at a lower daily dosage, it may be e.g. about 1/50 to ½, preferably 1/50 to 1/10, of the standard daily dosage of the S1 P receptor modulator or agonist.

In one embodiment of the invention, the total dosage of said S1 P receptor modulator or agonist as defined in the claims during the initial 3 to 6 days, preferably 4 or 5 days, most preferred 4 days, of treatment is increased incrementally from 4 to 12-fold, particularly about 10-fold, the standard daily dosage of said S1 P receptor modulator or agonist. For example, the loading dose may be 1; 1.5-2; 2-3; and 3-4 fold the standard daily dosage, on day 1, 2, 3 and 4, respectively.

According to the invention according to claim 1, the highest loading regimen dose instalment on the last day of the loading regimen, e.g. on day 4, is 4x the maintenance dose of the S1 P receptor modulator or agonist. The instalment doses on days 1, 2 and 3 of the loading regimen may be e.g. about ¼; ½; and ¾ of the highest instalment dose of the S1 P receptor modulator or agonist.

A particularly preferred dosage of the S1 P receptor modulator or agonist as defined in claim 1, e.g. the preferred S1 P receptor modulator FTY720, is e.g. 2-5, 5-10, 10-15 and 15-20 mg, e.g. a regimen of 2.5mg/5mg/7.5mg/l0mg or 5mg/10mg/15mg/20mg, respectively, during the initial period of 4 days. Thereafter the treatment is continued with the maintenance therapy, e.g. a daily dosage of 2.5 mg or 5 mg, or at a lower daily dosage, e.g. 0.1 to 0,5 mg.

In a further embodiment of the invention, a preferred loading regimen of a S1 P receptor agonist or modulator as defined in claim 1, e.g. the preferred S1P receptor modulator FTY720, may also be e.g. 0.5mg/1mg/1.5mg/2mg during the initial period of 4 days. Thereafter the treatment is continued with the maintenance therapy.

In a series of further specific or alternative embodiments, the present disclosure also provides:
1.1 (for reference) The use of a S1 P receptor modulator or agonist, e.g. FTY720, in the manufacture of a medication, whereby said medication is administered in such a way to a subject that a steady-state of the S1 P receptor modulator or agonist blood levels is attained in less than a week.
   The steady-state attained is such that the subject is sufficiently immunosuppressed, e.g. it shows no signs or symptoms of acute graft rejection or relapse or rebound of the autoimmune disease. During the initial 3 to 6 days, preferably 4 or 5 days, most preferred 4 days, of treatment the daily dosage of the S1 P receptor modulator or agonist is raised stepwise up to 3- to 21-fold the standard daily dosage of said S1 P receptor modulator or agonist and thereafter the treatment is continued with the standard daily dosage of said S1 P receptor modulator or agonist.
1.2 (for reference) The use of a S1 P receptor modulator or agonist, e.g. FTY720, in the manufacture of a medication, whereby said medication is administered in such a way to a subject that a steady-state of the S1 P receptor modulator or agonist blood levels is attained in less than a week, and thereafter the treatment is continued at a dosage lower than the standard daily dosage.
1.3. (as invention embodiment) The use of a S1P receptor modulator or agonist as defined in claim 1, e.g. FTY720, in the manufacture of a medication, whereby said medication is administered in such a way that during the initial 4 days of treatment the dosage of the S1 P receptor modulator or agonist is 1; 1.5-2; 2-3; and 3-4 fold the standard daily dosage, respectively, and thereafter the treatment is continued with the standard daily dosage of the S1 P receptor modulator or agonist, or at a lower daily dosage.
1.4 (as invention embodiment) The use of a S1 P receptor modulator or agonist as defined in claim 1, e.g. FTY720, in the manufacture of a medication, whereby said medication is administered in such a way that during the initial 4 days of treatment the dosage of the S1 P receptor modulator or agonist is ¼; ½; and ¾ of the highest instalment dose of the S1 P receptor modulator or agonist; and 4x the maintenance dose of the S1 P receptor modulator or agonist, respectively, and thereafter the treatment is continued with the maintenance dose or optionally with a lower daily dosage of the S1 P receptor modulator or agonist.
1.5 (for reference) The use of an S1 P receptor modulator or agonist, e.g. FTY720, in the manufacture of a medication, whereby said medication is administered in such a way that during the initial 3 to 6 days, preferably 4 or 5 days, most preferred 4 days, of treatment the dosage of said S1 P receptor modulator or agonist is raised so that in total the R-fold standard daily dosage of said S1 P receptor modulator or agonist is administered and thereafter the treatment is continued with the standard daily dosage of said S1 P receptor agonist or at a lower daily dosage.
1.6 (for reference) The use of an S1 P receptor modulator or agonist, e.g. FTY720, in the manufacture of a medication, whereby said medication is administered, after a loading regimen, at a daily dosage which is lower than the standard daily dosage.
1.7 (as invention embodiment) The use of FTY720 in the manufacture of a medication, whereby said medication is administered, after a loading regimen as defined in any one of the claims, at a daily dosage of 0.1 to 0.5 mg.
2. ((for reference) A method for inhibiting graft rejection or treating an autoimmune disease in a subject in need thereof, comprising administering to the subject a S1 P receptor modulator or agonist, e.g. FTY720, in such a pharmaceutically effective amount that a steady-state of the S1 P receptor modulator or agonist blood levels is attained in the subject in less than a week. Thereafter the treatment is continued with the standard daily dosage of said S1 P receptor modulator or agonist or at a lower daily dosage
2.1 (for reference) A method for producing a steady-state of S1 P receptor modulator or agonist blood levels in a subject in less than a week comprising administering during the initial 3 to 6 days, preferably 4 or 5 days, most preferred 4 days, an incremental daily dosage of up 3- to 21-fold the standard daily dosage of said S1 P receptor modulator or agonist.
2.2 (for reference) In a treatment method with a S1P receptor modulator or agonist, e.g. FTY720, the improvement being that the S1P receptor modulator or agonist is administered in such a way that during the initial 3 to 6 days, preferably 4 or 5 days, most preferred 4 days, of treatment the dosage is raised so that in total the R-fold standard daily dosage is administered. Thereafter the treatment is continued with the standard effective daily dosage or at a lower daily dosage.
2.3 (for reference) A method for providing prolonged transplant survival rates in a subject, whereby an S1 P receptor modulator or agonist is administered in such a way that during the initial 3 to 6 days, preferably 4 or 5 days, most preferred 4 days, of treatment the dosage is raised stepwise so that in total the R-fold standard daily dosage is administered and thereafter the treatment is continued with the standard daily dosage or at a lower daily dosage.
2.4 (for reference) A method for inhibiting graft rejection or treating an autoimmune disease in a subject in need thereof, comprising administering to the subject, after a loading regimen, a S1P receptor modulator or agonist, e.g. FTY720, at a daily dosage which is lower than the standard daily dosage.
2.5 (for reference) A method for treating an autoimmune disease in a subject in need thereof, comprising administering to the subject, after a loading regimen, a daily dosage of FTY720 of about 0.1 to 0.5mg.
3. ((for reference) A kit containing daily units of medication of an S1P receptor modulator or agonist, e.g. FTY720, of varying daily dosage, whereby the daily dosage of said S1P receptor modulator or agonist for the initial 3 to 6 days, preferably 4 or 5 days, most preferred 4 days, of treatment is incrementally increased so that the total amount present in the daily units corresponds to the R-fold standard daily dosage of said S1P receptor modulator or agonist for this initial time period.
3.1. (as invention embodiment) A kit containing daily units of medication of an S1P receptor modulator or agonist as defined in claim 1, e.g. FTY720, of varying daily dosage, whereby the daily dosage of S1P receptor modulator or agonist for the initial 4 days of treatment is 1; 1.5-2; 2-3; and 3-4 fold the standard daily dosage, respectively. The kit may further comprise units for the standard daily dosage of the S1P receptor modulator or agonist as defined in claim 1, e.g. FTY720, or for the subsequent treatment with a lower daily dosage. The kit may also contain instructions for use.
3.2 (for reference) A kit containing daily units of medication of an S1P receptor modulator or agonist, e.g. FTY720, of varying daily dosage, whereby the daily dosage of S1P receptor modulator or agonist for the initial 4 days of treatment is ¼; ½; and ¾ of the highest instalment dose of the S1P receptor modulator or agonist; and 4x the maintenance dose of the S1P receptor modulator or agonist, respectively. The kit may further comprise units for the standard daily dosage of the S1P receptor modulator or agonist, e.g. FTY720, or for the subsequent treatment with a lower daily dosage. The kit may also contain instructions for use.

The loading regimen of S1P receptor modulator or agonist which is administered to the subject according to the invention may be given at the beginning of an autoimmune disease therapy, or after an interruption of S1P receptor modulator or agonist therapy.

Utility of an S1P receptor modulator or agonist dosage regimen in treating diseases and conditions as hereinabove specified may be demonstrated in standard animal or clinical tests, e.g. in accordance with the methods described hereinafter.

### 2-Phase Loading Regimen-Study:

Initial baseline (Day -2): on Day -2, subjects enter the study center at least 12-hours prior to dosing for verification of inclusion/exclusion criteria and baseline assessments.

Placebo run-in (Day -1): On Day -1, subjects receive a single, placebo dose of FTY720

FTY720 treatment (Days 1-7): All subjects receive FTY720 once daily for 7 consecutive days as follows,
Day 1: Subjects receive a single 5 mg FTY720 oral dose at the exact time the Day -1 dose was administered.
Days 2-4: Subject receive a single 10 mg FTY720 oral dose on Day 2, a single 15 mg FTY720 oral dose on Day 3, and a single 20 mg FTY720 oral dose on Day 4, in order to achieve the FTY720 steady-state concentration typically measured in patients on chronic dosing of FTY720 5 mg qd.
Day 5-7: Subjects receive single 5 mg FTY720 oral doses once daily.

Pharmacokinetic, pharmacodynamic and safety assessments are performed at specified times during the multiple-dose study. Subjects are released from the study center approximately 24 hours after the last drug administration on Day 7, after the safety evaluations have been completed (i.e., Day 8).

### • Analytes, media and methods:

FTY720 is measured in whole blood using LC/MS/MS (LLOQ = 0.080 ng/mL)
- PK evaluations: Noncompartmental analysis to derive tmax, Cmax, AUC(0-24) on day 1. Peak and trough concentrations are summarized from days 2 through 7 to estimate drug accumulation and attainment of steady state.

### Lymphocyte assessment

Blood samples for absolute lymphocyte counts is collected at screening, at initial baseline (Day -2), Day 1 (6h postdose), Day 3 (predose), Day 5 (predose) and Day 7 (predose).

The samples are analyzed for pharmacodynamics.

Above procedure may be repeated and the patients are then treated Day 5 and followings with a daily maintenance dose of 0.5mg/kg. The patients have lower steady-state blood levels.

Above procedure may be repeated with following loading treatments:
1. Day 1: Subjects receive a single 2.5 mg FTY720 oral dose at the exact time the Day - 1 dose was administered.
   Days 2-4: Subject receive a single 5 mg FTY720 oral dose on Day 2, a single 7.5 mg FTY720 oral dose on Day 3, and a single 10 mg FTY720 oral dose on Day 4, in order to achieve the FTY720 steady-state concentration typically measured in patients on chronic dosing of FTY720 2.5 mg qd.
   Day 5-7 and following: Subjects receive single 2.5 mg FTY720 oral doses once daily.
2. Day 1: Subjects receive a single 1.25 mg FTY720 oral dose at the exact time the Day -1 dose was administered.
   Days 2-4: Subject receive a single 2.5 mg FTY720 oral dose on Day 2, a single 3.75 mg FTY720 oral dose on Day 3, and a single 5 mg FTY720 oral dose on Day 4, in order to achieve the FTY720 steady-state concentration typically measured in patients on chronic dosing of FTY720 1.25 mg qd.
   Day 5-7 and following: Subjects receive single 1.25 mg FTY720 oral doses once daily.

## Claims

1. An S1P receptor modulator or agonist for use in a method of treating an autoimmune disease or disorder,
wherein the daily dosage of said S1P receptor modulator or agonist, which is selected from 2-amino-2-[2-(4-octylphenyl) ethyl]propane-1,3-diol in free form or in a pharmaceutically acceptable salt form and from the compound
of the formula VIa 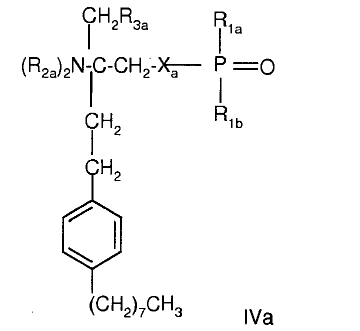 wherein R₂ₐ is H, R₃ₐ is OH, Xₐ is O, R₁ₐ and R_{1b} are OH,
is increased stepwise in a loading regimen with a highest loading regimen dose instalment of 4x or 4-fold the maintenance (standard) dose of the S1 P receptor modulator or agonist during the initial period of treatment of the first 3 days (days 1-3) to the first 6 days of treatment (days 1-6), wherein
the standard daily dosage is the dosage necessary for a stable blood level concentration of the medication providing effective treatment, and thereafter the treatment is continued with the standard daily dosage or with a lower daily dosage.

2. The S1 P receptor modulator or agonist for use according to claim 1, wherein the loading regimen is such that a steady-state of the S1P receptor modulator or agonist blood levels is attained in less than a week.

3. An S1P receptor modulator or agonist for use in a method of treating an autoimmune disease or disorder,
wherein the daily dosage of said S1 P receptor modulator or agonist, which is selected from 2-amino-2-[2-(4-octylphenyl) ethyl]propane-1,3-diol in free form or in a pharmaceutically acceptable salt form and from the compound of the formula VIa 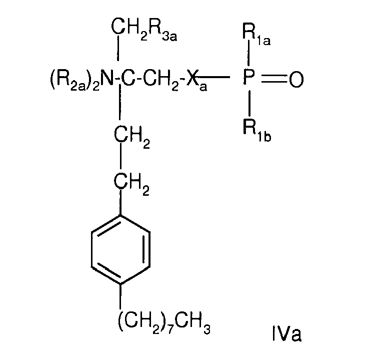 wherein R₂ₐ is H, R₃ₐ is OH, Xₐ is O, R₁ₐ and R_{1b} are OH,
is increased incrementally to 4 to 12-fold the standard daily dosage of said S1 P receptor modulator or agonist such that a steady state of the S1 P receptor modulator or agonist blood levels is attained in less than a week and wherein the standard daily dosage is the dosage necessary for a stable blood level concentration of the medication providing effective treatment, and thereafter the treatment is continued with the standard daily dosage or with a lower dosage.

4. The S1 P receptor modulator or agonist for use according to claim 1 or claim 2 or claim 3, wherein the initial period of treatment is 4 days.

5. The S1P receptor modulator or agonist for use according to claim 1 or 2, wherein the S1P receptor modulator or agonist is 2-amino-2-[2-(4-octylphenyl) ethyl]propane-1,3-diol in free form or in a pharmaceutically acceptable salt form and is administered, after the loading regimen, at a daily dosage of 0.1 to 0.5 mg.

6. The S1P receptor modulator or agonist for use according to claim 1 or 2, wherein a loading dose of 1; 1,5-2; 2-3; and 3-4-fold of the standard daily dosage is used on days 1, 2, 3 and 4, respectively.

7. The S1P receptor modulator or agonist for use according to claim 1 or 2, wherein the instalment doses of the loading regimen on days 1, 2 and 3 are ¼; ½; and ¾ of the highest instalment dose of the S1P receptor modulator or agonist.

8. The S1P receptor modulator or agonist for use according to claim 1 or 2, wherein the S1P receptor modulator dosage is 2-5, 5-10, 10-15 and 15-20 mg during the initial period of 4 days.

9. The S1P receptor modulator or agonist for use according to claim 1 or 2, wherein the regimen is 2.5 mg/5mg/7.5mg/10mg during the first 4 days and thereafter treatment is continued after the initial period of 4 days with a maintenance therapy at a daily dosage of 2.5 mg or 5 mg, or at a lower daily dosage of 0.1 to 0.5 mg.

10. The S1P receptor modulator or agonist for use according to claim 9, wherein the regimen is 2.5 mg/5mg/7.5mg/10mg during the first 4 days and thereafter treatment is continued after the initial period of 4 days with dosage of 0.1 to 0.5 mg.

11. The S1P receptor modulator or agonist for use according to claim 1 or 2, wherein the regimen is 5 mg/10mg/15mg/20mg during the first 4 days and therafter treatment is continued after the initial period of 4 days with a maintenance therapy at a daily dosage of 2.5 or 5 mg.

12. The S1P receptor modulator or agonist for use according to any one of claims 1 to 11, wherein the S1P receptor modulator or agonist is 2-amino-2-[2-(4-octylphenyl) ethyl]propane-1,3-diol in free form or in a pharmaceutically acceptable salt form

13. The S1P receptor modulator or agonist for use in a method of treating an autoimmune disease or disorder according to any one of claims 1 to 11, wherein the S1P receptor modulator or agonist is 2-amino-2-[2-(4-octylphenyl) ethyl]propane-1,3-diol hydrochloride.

14. The S1P receptor modulator or agonist for use in a method of treating an autoimmune disease or disorder in a subject in need thereof according to any one of the preceding claims wherein the disease or disorder is multiple sclerosis, lupus nephritis, rheumatoid arthritis, inflammatory bowel diseases or psoriasis.

15. A kit for use according to claim 1 containing daily units of medication of an S1P receptor modulator or agonist of varying daily dosage, whereby the daily dosage of S1P receptor modulator or agonist for the initial 4 days of treatment is 1-;1.5-2-; 2-3-; and 3-4- fold the standard daily dosage, respectively, and optionally units for the subsequent treatment with the standard daily dosage of the S1 P receptor modulator or agonist , or with a lower daily dosage, and wherein the standard daily dosage is the dosage necessary for a stable blood level concentration of the medication providing effective treatment, wherein the S1P receptor modulator or agonist is selected from 2-amino-2-[2-(4-octylphenyl) ethyl]propane-1,3-diol in free form or in a pharmaceutically acceptable salt form and from the compound of the formula VIa 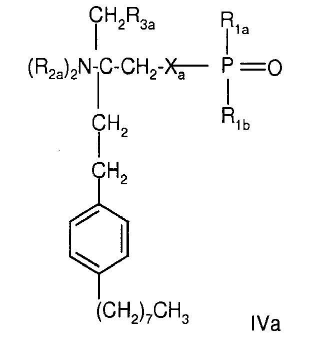 wherein R₂ₐ is H, R₃ₐ is OH, Xₐ is O, R₁ₐ and R_{1b} are OH.

16. The kit according to claim 15 for use according to claim 1, wherein the S1P receptor modulator or agonis2-amino-2-[2-(4-octylphenyl) ethyl]propane-1,3-diol in free form or in a pharmaceutically acceptable salt form.

## Patentansprüche

1. S1P-Rezeptor-Modulator oder -Agonist zur Verwendung bei einem Verfahren zur Behandlung einer Autoimmunkrankheit oder -störung,
wobei die Tagesdosis des S1P-Rezeptor-Modulators oder -Agonisten, der aus 2-Amino-2-[2-(4-octylphenyl)ethyl]propan-1,3-diol in freier Form oder in Form pharmazeutisch akzeptablen Salzes und aus der Verbindung der Formel IVa 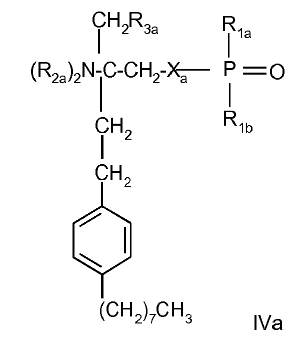 wobei R₂ₐ H ist, R₃ₐ OH ist, Xₐ O ist, R₁ₐ und R_{1b} OH sind,
ausgewählt ist, in einem Aufsättigungsregime mit einem höchsten Aufsättigungsregime-Dosisteil vom 4x- bzw. 4-Fachen der Erhaltungsdosis (Standarddosis) des S1P-Rezeptor-Modulators oder -Agonisten während der ersten Phase der Behandlung der ersten 3 Tage (Tag 1 bis 3) bis zu den ersten 6 Tagen der Behandlung (Tag 1 bis 6) schrittweise erhöht wird, wobei die Standard-Tagesdosis die Dosis ist, die für eine stabile Blutspiegelkonzentration des Medikaments erforderlich ist, die eine wirksame Behandlung bereistellt, und die Behandlung danach mit der Standard-Tagesdosis oder mit einer niedrigeren Tagesdosis fortgesetzt wird.

2. S1 P-Rezeptor-Modulator oder -Agonist zur Verwendung nach Anspruch 1, wobei das Aufsättigungsregime derart ist, dass ein Steady-State der S1 P-Rezeptor-Modulator- oder -Agonisten-Blutspiegel in weniger als einer Woche erhalten wird.

3. S1P-Rezeptor-Modulator oder -Agonist zur Verwendung bei einem Verfahren zur Behandlung einer Autoimmunkrankheit oder -störung,
wobei die Tagesdosis des S1P-Rezeptor-Modulators oder -Agonisten, der aus 2-Amino-2-[2-(4-octylphenyl)ethyl]propan-1,3-diol in freier Form oder in Form pharmazeutisch akzeptablen Salzes und aus der Verbindung der Formel IVa 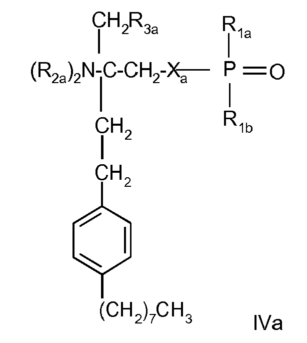 wobei R₂ₐ H ist, R₃ₐ OH ist, Xₐ O ist, R₁ₐ und R_{1b} OH sind,
ausgewählt ist, schrittweise auf das 4- bis 12-Fache der Standard-Tagesdosis des S1 P-Rezeptor-Modulators oder -Agonisten erhöht wird, so dass ein Steady-State der S1 P-Rezeptor-Modulator- oder -Agonisten-Blutspiegel in weniger als einer Woche erzielt wird, und wobei die Standard-Tagesdosis die Dosis ist, die für eine stabile Blutspiegelkonzentration des Medikaments erforderlich ist, die eine wirksame Behandlung bereitstellt, und die Behandlung danach mit der Standard-Tagesdosis oder mit einer niedrigeren Dosis fortgesetzt wird.

4. S1P-Rezeptor-Modulator oder -Agonist zur Verwendung nach Anspruch 1 oder Anspruch 2 oder Anspruch 3, wobei die erste Phase der Behandlung 4 Tage ist.

5. S1 P-Rezeptor-Modulator- oder -Agonist zur Verwendung nach Anspruch 1 oder 2, wobei der S1 P-Rezeptor-Modulator oder -Agonist 2-Amino-2-[2-(4-octylphenyl)ethyl]propan-1,3-diol in freier Form oder in Form pharmazeutisch akzeptablen Salzes ist und nach dem Aufsättigungsregime in einer Tagesdosis von 0,1 bis 0,5 mg verabreicht wird.

6. S1P-Rezeptor-Modulator oder -Agonist zur Verwendung nach Anspruch 1 oder 2, wobei eine Aufsättigungsdosis von dem 1-; 1,5- bis 2; 2- bis 3-; und 3- bis 4-Fachen der Standard-Tagesdosis an Tag 1, 2, 3 bzw. 4 verwendet wird.

7. S1 P-Rezeptor-Modulator oder -Agonist zur Verwendung nach Anspruch 1 oder 2, wobei die Teildosen des Aufsättigungsregimes an Tag 1, 2 und 3 1/4; 1/2; und 3/4 der höchsten Teildosis des S1P-Rezeptor-Modulators oder -Agonisten ist.

8. S1P-Rezeptor-Modulator oder -Agonist zur Verwendung nach Anspruch 1 oder 2, wobei die Dosis des S1P-Rezeptor-Modulators oder -Agonisten während der ersten Phase von 4 Tagen 2 bis 5, 5 bis 10, 10 bis 15 und 15 bis 20 mg beträgt.

9. S1 P-Rezeptor-Modulator oder -Agonist zur Verwendung nach Anspruch 1 oder 2, wobei das Regime 2,5 mg/5 mg/7,5 mg/10 mg während der ersten 4 Tage ist und die Behandlung danach nach der ersten Phase von 4 Tagen mit einer Erhaltungstherapie in einer Tagesdosis von 2,5 oder 5 mg oder mit einer niedrigeren Tagesdosis von 0,1 bis 0,5 mg fortgesetzt wird.

10. S1 P-Rezeptor-Modulator oder -Agonist zur Verwendung nach Anspruch 9, wobei das Regime 2,5 mg/5 mg/7,5 mg/10 mg während der ersten 4 Tage ist und die Behandlung danach nach der ersten Phase von 4 Tagen mit einer Dosis von 0,1 bis 0,5 mg fortgesetzt wird.

11. S1P-Rezeptor-Modulator oder -Agonist zur Verwendung nach Anspruch 1 oder 2, wobei das Regime 5 mg/10mg/15mg/20mg während der ersten 4 Tage ist und die Behandlung danach nach der ersten Phase von 4 Tagen mit einer Erhaltungstherapie in einer Tagesdosis von 2,5 bis 5 mg fortgesetzt wird.

12. S1 P-Rezeptor-Modulator- oder -Agonist zur Verwendung nach einem der Ansprüche 1 bis 11, wobei der S1P-Rezeptor-Modulator oder -Agonist 2-Amino-2-[2-(4-octylphenyl)ethyl]propan-1,3-diol in freier Form oder in Form pharmazeutisch akzeptablen Salzes ist.

13. S1 P-Rezeptor-Modulator- oder -Agonist zur Verwendung bei einem Verfahren zur Behandlung einer Autoimmunkrankheit oder -störung nach einem der Ansprüche 1 bis 11, wobei der S1P-Rezeptor-Modulator oder -Agonist 2-Amino-2-[2-(4-octylphenyl)ethyl]propan-1,3-diolhydrochlorid ist.

14. S1 P-Rezeptor-Modulator oder -Agonist zur Verwendung bei einem Verfahren zur Behandlung einer Autoimmunkrankheit oder -störung bei einem Individuum, das einer solchen bedarf, nach einem der vorstehenden Ansprüche, wobei die Krankheit oder der Zustand multiple Sklerose, Lupusnephritis, rheumatoide Arthritis, entzündliche Darmkrankheiten oder Psoriasis ist.

15. Kit zur Verwendung nach Anspruch 1, der Medikament-Tageseinheiten eines S1P-Rezeptor-Modulators oder -Agonisten variierender Tagesdosis, wobei die Tagesdosis des S1P-Rezeptor-Modulators oder -Agonisten für die ersten 4 Tage der Behandlung das 1-; 1,5- bis 2-; 2- bis 3-; bzw. 3- bis 4-Fache der Standard-Tagesdosis ist, und optional Einheiten für die darauf folgende Behandlung mit der Standard-Tagesdosis des S1 P-Rezeptor-Modulators oder -Agonisten oder mit einer niedrigeren Tagesdosis enthält, und wobei die Standard-Tagesdosis die Dosis ist, die für eine stabile Blutspiegelkonzentration des Medikaments erforderlich ist, die eine wirksame Behandlung bereitstellt, wobei der S1 P-Rezeptor-Modulator oder -Agonist aus 2-Amino-2-[2-(4-octylphenyl)ethyl]propan-1,3-diol in freier Form oder in Form pharmazeutisch akzeptablen Salzes oder aus der Verbindung der Formel IVa 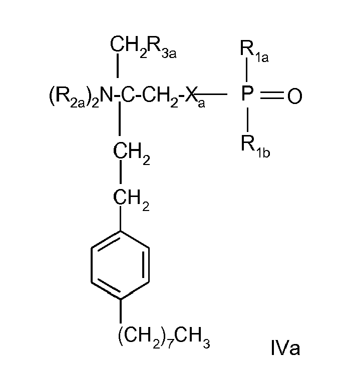 wobei R₂ₐ H ist, R₃ₐ OH ist, Xₐ O ist, R₁ₐ und R_{1b} OH sind, ausgewählt ist.

16. Kit nach Anspruch 15 zur Verwendung nach Anspruch 1, wobei der S1P-Rezeptor-Modulator- oder -Agonist 2-Amino-2-[2-(4-octylphenyl)ethyl]propan-1,3-diol in freier Form oder in Form pharmazeutisch akzeptablen Salzes ist.

## Revendications

1. Modulateur ou agoniste des récepteurs de S1P pour l'utilisation dans une méthode de traitement d'une maladie auto-immune ou d'un trouble auto-immun,
dans lequel le dosage quotidien dudit modulateur ou agoniste des récepteurs de S1P, qui est choisi parmi le 2-amino-2-[2-(4-octylphényl) éthyl]propane-1,3-diol sous forme libre ou sous forme de sel pharmaceutiquement acceptable et le composé de la formule IVa : 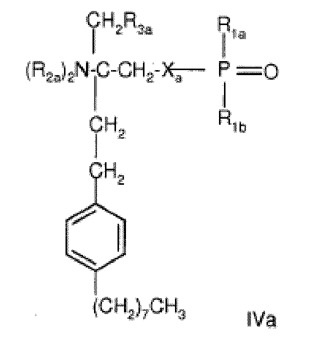 dans laquelle R₂ₐ représente H, R₃ₐ représente OH, Xₐ représente O, R₁ₐ et R_{1b} représentent OH,
est augmenté par étapes dans un régime de charge avec une fraction de dose de régime de charge la plus élevée de 4x ou 4 fois la dose (standard) d'entretien du modulateur ou agoniste des récepteurs de S1P pendant la période initiale de traitement des 3 premiers jours (jours 1-3) aux 6 premiers jours de traitement (jours 1-6), le dosage quotidien standard étant le dosage nécessaire pour une concentration de taux sanguin stable du médicament assurant un traitement efficace, puis le traitement est poursuivi par le dosage quotidien standard ou par un dosage quotidien inférieur.

2. Modulateur ou agoniste des récepteurs de S1P pour l'utilisation selon la revendication 1, dans lequel le régime de charge est tel qu'un état stable des taux sanguins du modulateur ou agoniste des récepteurs de S1P est atteint en moins d'une semaine.

3. Modulateur ou agoniste des récepteurs de S1P pour l'utilisation dans une méthode de traitement d'une maladie auto-immune ou d'un trouble auto-immun,
dans lequel le dosage quotidien dudit modulateur ou agoniste des récepteurs de S1P, qui est choisi parmi le 2-amino-2-[2-(4-octylphényl)éthyl]propane-1,3-diol sous forme libre ou sous forme de sel pharmaceutiquement acceptable et le composé de la formule IVa : 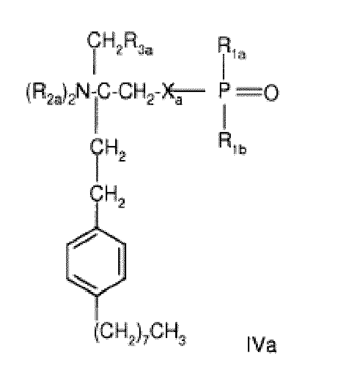 dans laquelle R₂ₐ représente H, R₃ₐ représente OH, Xₐ représente O, R₁ₐ et R_{1b} représentent OH,
est augmenté par incréments à 4 à 12 fois le dosage quotidien standard dudit modulateur ou agoniste des récepteurs de S1P de telle sorte qu'un état stable des taux sanguins du modulateur ou de l'agoniste des récepteurs de S1P est atteint en moins d'une semaine, et dans lequel le dosage quotidien standard est le dosage nécessaire pour une concentration de taux sanguin stable du médicament assurant un traitement efficace, puis le traitement est poursuivi par le dosage quotidien standard ou par un dosage inférieur.

4. Modulateur ou agoniste des récepteurs de S1P pour l'utilisation selon la revendication 1 ou la revendication 2 ou la revendication 3, dans lequel la période initiale de traitement est de 4 jours.

5. Modulateur ou agoniste des récepteurs de S1P pour l'utilisation selon l'une des revendications 1 ou 2, dans lequel le modulateur ou agoniste des récepteurs de S1P est le 2-amino-2-[2-(4-octylphényl)éthyl]propane-1,3-diol, sous forme libre ou sous forme de sel pharmaceutiquement acceptable et est administré, après le régime de charge, à un dosage quotidien de 0,1 à 0,5 mg.

6. Modulateur ou agoniste des récepteurs de S1P pour l'utilisation selon l'une des revendications 1 ou 2, dans lequel une dose de charge de 1 ; 1,5-2 ; 2-3 ; et 3-4 fois le dosage quotidien standard est utilisée aux jours 1, 2, 3 et 4, respectivement.

7. Modulateur ou agoniste des récepteurs de S1P pour l'utilisation selon l'une des revendications 1 ou 2, dans lequel les fractions de dose du régime de charge aux jours 1, 2 et 3 sont ¼ ; ½ et ¾ de la fraction de dose la plus élevée du modulateur ou agoniste des récepteurs de S1P.

8. Modulateur ou agoniste des récepteurs de S1P pour l'utilisation selon l'une des revendications 1 ou 2, dans lequel le dosage du modulateur des récepteurs de S1P est 2-5, 5-10, 10-15 et 15-20 mg pendant la période initiale de 4 jours.

9. Modulateur ou agoniste des récepteurs de S1P pour l'utilisation selon l'une des revendications 1 ou 2, dans lequel le régime est de 2,5 mg/5 mg/7,5 mg/10 mg pendant les 4 premiers jours, puis le traitement est poursuivi après la période initiale de 4 jours par une thérapie d'entretien à un dosage quotidien de 2,5 mg ou 5 mg, ou à un dosage quotidien inférieur de 0,1 à 0,5 mg.

10. Modulateur ou agoniste des récepteurs de S1P pour l'utilisation selon la revendication 9, dans lequel le régime est de 2,5 mg/5 mg/7,5 mg/10 mg pendant les 4 premiers jours, puis le traitement est poursuivi après la période initiale de 4 jours par un dosage de 0,1 à 0,5 mg.

11. Modulateur ou agoniste des récepteurs de S1P pour l'utilisation selon l'une des revendications 1 ou 2, dans lequel le régime est de 5 mg/10 mg/15 mg/20 mg pendant les 4 premiers jours, puis le traitement est poursuivi après la période initiale de 4 jours par une thérapie d'entretien à un dosage quotidien de 2,5 ou 5 mg.

12. Modulateur ou agoniste des récepteurs de S1P pour l'utilisation selon l'une quelconque des revendications 1 à 11, dans lequel le modulateur ou agoniste des récepteurs de S1P est le 2-amino-2-[2-(4-octylphényl) éthyl]propane-1,3-diol sous forme libre ou sous forme de sel pharmaceutiquement acceptable.

13. Modulateur ou agoniste des récepteurs de S1P pour l'utilisation dans une méthode de traitement d'une maladie auto-immune ou d'un trouble auto-immun selon l'une quelconque des revendications 1 à 11, dans lequel le modulateur ou agoniste des récepteurs de S1P est le chlorhydrate du 2-amino-2-[2-(4-octylphényl) éthyl]propane-1,3-diol.

14. Modulateur ou agoniste des récepteurs de S1P pour l'utilisation dans une méthode de traitement d'une maladie auto-immune ou d'un trouble auto-immun chez un sujet en ayant besoin, selon l'une quelconque des revendications précédentes, dans lequel la maladie ou le trouble est la sclérose en plaques, la néphropathie lupique, la polyarthrite rhumatoïde, les maladies inflammatoires de l'intestin ou le psoriasis.

15. Trousse pour l'utilisation selon la revendication 1 contenant des unités quotidiennes de médicament d'un modulateur ou agoniste des récepteurs de S1P de dosage quotidien variable, ce par quoi le dosage quotidien du modulateur ou agoniste des récepteurs de S1P pendant les 4 jours initiaux de traitement est 1 ; 1,5-2 ; 2-3 ; et 3-4 fois le dosage quotidien standard, respectivement, et facultativement des unités pour le traitement ultérieur par le dosage quotidien standard du modulateur ou agoniste des récepteurs de S1P, ou par un dosage quotidien inférieur, et dans laquelle le dosage quotidien standard est le dosage nécessaire pour une concentration de taux sanguin stable du médicament assurant un traitement efficace, le modulateur ou agoniste des récepteurs de S1P est choisi parmi le 2-amino-2-[2-(4-octylphényl)éthyl]propane-1,3-diol sous forme libre ou sous forme de sel pharmaceutiquement acceptable et le composé de la formule IVa : 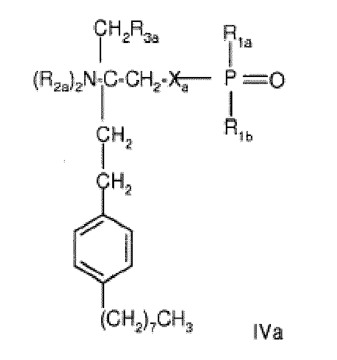 dans laquelle R₂ₐ représente H, R₃ₐ représente OH, Xₐ représente O, R₁ₐ et R_{1b} représentent OH.

16. Trousse selon la revendication 15 pour l'utilisation selon la revendication 1, dans laquelle le modulateur ou agoniste des récepteurs de S1P est le 2-amino-2-[2-(4-octylphényl)éthyl]propane-1,3-diol sous forme libre ou sous forme d'un sel pharmaceutiquement acceptable.
